# EUROPÄISCHE PATENTANMELDUNG

(11) **EP 2 261 194 A2**
(43) Veröffentlichungstag der Anmeldung: **15.12.2010**
(21) Anmeldenummer: 10183630.2
(22) Anmeldetag: 15.12.2003
(51) Int. Cl.: C04B 35/486, A61K 6/06

(54) **Fräskeramiken aus Metalloxid-Pulvern mit bimodaler Korngrößenverteilung**

(30) Priorität: 30.12.2002 DE 10261720
(62) Teilanmeldung aus: 03028804.7
(71) Anmelder: Chemichl AG, 9490 Vaduz (LI); Meyer, Gerhard, Prof., Dr., 46483 Wesel (DE)
(72) Erfinder: Meyer, Gerhard, 46483 Wesel (DE); Conrad, Thomas, 44287 Dortmund (DE)
(74) Vertreter: Jostarndt, Hans-Dieter

(57) **Zusammenfassung**

Die vorliegende Erfindung betrifft Metalloxid-Pulver mit bimodaler Korngrößenverteilung, aus diesen Metalloxid-Pulvern herstellbare Keramiken, insbesondere Fräskeramiken zum Einsatz in der Dentaltechnik, Verfahren zur Herstellung der Metalloxid-Pulver und der Keramiken, die Verwendung nanoskaliger Metalloxid-Pulver zur Herstellung der Metalloxid-Pulver und der Keramiken und Dentalkeramikprodukte.

## Beschreibung

Die vorliegende Erfindung betrifft Metalloxid-Pulver mit bimodaler Korngrößenverteilung bzw. bimodale Keramik-Binder-Werkstoffverbünde, aus diesen Metalloxid-Pulvern (Verbünden) herstellbare Keramiken, insbesondere Fräskeramiken zum Einsatz in der Dentaltechnik, Verfahren zur Herstellung der Metalloxid-Pulver und der Keramiken, die Verwendung nanoskaliger Metalloxid-Pulver zur Herstellung der Metalloxid-Pulver und der Keramiken und Dentalkeramikprodukte.

Keramiken aus Metalloxid-Pulvern, besonders Al₂O₃, werden auf Grund ihrer mechanischen Belastbarkeit und Biokompatibilität in der Dentaltechnik seit geraumer Zeit eingesetzt. In jüngerer Zeit findet auch teilstabilisiertes ZrO₂ Berücksichtigung, da es wegen seiner polymorphen Erscheinungsform eine gegenüber dem Al₂O₃ gesteigerte mechanische Festigkeit aufweist. Die Verarbeitung dieser Keramiken erfolgt hierbei mittels einer Fräse, wobei entweder der Grünkörper, ein Vorsinterkörper, ein endgesinterter poröser Körper (mit anschließender Glasinfiltration) oder ein endgesinterter Voll-Keramikkörper spanend bearbeitet werden. Zu Beginn werden die Metalloxid-Pulver zunächst unter Druck verdichtet. Hierfür kommen in der Regel kalt isostatische oder uniaxiale Pressverfahren in Betracht, wobei uniaxiales Pressen auf Grund des unvermeidlichen Dichtegradienten gegenüber dem CIP-Verfahren keine homogene Dichte ermöglicht. Alternativ zu dieser Herstellung von Grünkörpern werden in der Dentalindustrie, z.B. von der Fa. Metoxit heiß isostatisch behandelte Keramikblöcke geliefert. Hierbei wird das keramische Ausgangspulver simultan verdichtet und gesintert. Hierdurch erreicht man die höchste Verdichtung, die z.B. beim 3 mol-% Y₂O₃ dotierten ZrO₂ mit 6,065 g/cm³ nahe der theoretischen Dichte liegt. Dieses Verfahren ist jedoch sehr kostenintensiv und liefert Keramikblöcke, welche auf Grund der hohen Dichte z.B. auf einer Dentalfräse der Fa. DCS in einem bis zu sechsstündigen Fräsvorgang zu einer fertigen dreigliedrigen Brücke bearbeitet werden kann. Dentsply Degussa Dental bietet ein alternatives Verfahren an. Hierbei wird der verdichtete Grünkörper zunächst, unter Berücksichtigung einer Schrumpfzugabe spanend bearbeitet und anschließend endgesintert. Ein Fräsen des Grünkörpers ist jedoch auf Grund der relativ geringen Gründichte monomodaler Pulver nicht unproblematisch, da es häufig während der spanenden Bearbeitung zu Ausbrüchen kommen kann. Auch ein Versand der Grünkörper an die mit ihrer Verarbeitung beschäftigten Dentallabore ist auf Grund der nicht optimalen Gründichte problematisch. Weiterhin ist auf Grund der großen Schrumpfung die Einstellung einer, für die dentalen Anforderungen noch tolerierbaren, Abweichung vom isotropen Schrumpf nicht unproblematisch. Des Weiteren erweisen sich die hohe Sintertemperatur und die lange Sinterdauer für den praktischen Einsatz als nachteilig, da es z.B. zu verstärkter Beanspruchung und verstärktem thermischen Verschleiß von Heizelementen führt, oder der Einsatz teurer Ofentypen erforderlich wird.

Die geringere Schrumpfung des erfindungsgemäßen bimodalen Metalloxid-Pulvers führt auch zu einer besseren Einstellung einer annähernd isotropen Schrumpfung insbesondere von Freiformflächen. Ferner ergibt sich durch die größere Packungsdichte eine geringere Schrumpfung, wodurch die Grünkörper weniger Raum beim Transport sowie im Sinterofen beanspruchen. Auch kann bei Bedarf die Sintertemperatur abgesenkt werden, ohne dass die für die dentale Anwendung erforderliche Festigkeit des Produktes unterschritten wird.

Das von der Fa. Vita praktizierte In-Ceram-Verfahren besteht in der Herstellung endgesinterter poröser Keramikblöcke, welche auch von Dentalfräsen geringer Leistung spanend bearbeitet werden können. Um die für den Gebrauch nötige Festigkeit zu erreichen, wird der poröse Körper mit Lanthanglas infiltriert, wobei die Infiltrationstemperatur unterhalb der Sintertemperatur des porösen endgesinterten Keramikkörpers liegt und somit eine Schrumpfung nahezu vermieden wird. Problematisch hierbei ist sowohl die recht geringe Festigkeit des porösen endgesinterten Keramikkörpers (eingeschränkte Handhabung) als auch die nicht optimale Festigkeit des Keramik-Glasverbundes nach der Infiltration. Durch Dotierung mit nanoskaligem Keramik-Pulver gelingt eine Festigkeitssteigerung des als Gerüst fungierenden porösen endgesinterten Keramikblocks.

Zur Absenkung der Sintertemperatur bei der Herstellung von Dental-Fräskeramiken ist vorgeschlagen worden, anstelle der üblichen Metalloxid-Pulver mit Korngrößen größer 1 µm sogenannte nanoskalige Metalloxid-Pulver zu verwenden, d.h. Metalloxid-Pulver, deren durchschnittliche Korngrößen im Nanometer-Bereich liegen. Jedoch haben sich Handhabung und Verarbeitung dieser "Nanopulver" in der Praxis als schwierig erwiesen. So kann es wegen ihrer hohen Sinteraktivität zu unerwünschter Agglomeration und verstärktem Kornwachstum kommen. Weiterhin erweist sich die Formgebung wegen der geringen Schütt- bzw. Klopfdichte häufig als schwierig. Somit ist der für die Einstellung eines nanoskaligen Gefüges erforderliche technische Aufwand so hoch, dass sich am Dentalmarkt nicht im ausreichenden Maß Erlöse erzielen lassen. Des weiteren scheitert der Einsatz reiner nanoskaliger Metalloxidpulver an deren momentan noch sehr hohen Herstellungskosten. Ein besonderer Effekt eines nanoskaligen Gefüges erweist sich jedoch besonders für die Dentalindustrie als vorteilhaft. Liegt der Korngrenzenbereich des Gefüges der gesinterten Probe unter 1/20 der Wellenlänge des sichtbaren Lichtes, so liegt Transparenz vor. In der Praxis führt u.a. auch eine Korngröße, welche unterhalb der Wellenlänge des sichtbaren Lichtes liegt, zu einer mehr oder weniger ausgeprägten Transluzenz. Diese Transluzenz wird auch durch besonders chemisch reine Ausgansmaterialien verbessert, da sich z. B. keine Verunreinigungen an den Korngrenzen ablagern können. Eine erhöhte Transluzenz der Gerüstkeramik führt in der dentalen Praxis zu einem geringeren Schichtbedarf an Verblendkeramiken, was einerseits zu einer leichteren Einstellung einer optimalen Ästhetik führt und andererseits einen geringeren Abschliff der für den Halt einer dentalen Brücke benötigten natürlichen Zähnen zur Folge hat.

Es ist daher eine Aufgabe der vorliegenden Erfindung, ein Metalloxid-Pulver bzw. einen Keramik-Binder-Verbund für die Anwendung in der Dentalindustrie bereitzustellen, die einerseits eine möglichst optimale Resistenz gegenüber Transport- und Handhabungsschäden aufweisen und sich andererseits zur Herstellung einer Keramik eignen, die nach Möglichkeit vor ihrer abschließenden Sinterung einer optimalen Fräsbearbeitung (optimale Gründichte) unterzogen werden kann, und hierbei - auch bei erheblicher Herabsetzung der Sintertemperatur und erheblicher Verkürzung der Sinterdauer - eine ausreichende Festigkeit und eine möglichst verbesserte Transluzenz aufweist.

Gelöst wird diese Aufgabe durch ein bimodales Metalloxid-Pulver (bimodaler Keramik-Binder-Verbund) , umfassend
(a) ein erstes Metalloxid-Pulver; und
(b) ein zweites, nanoskaliges hochsinteraktives Metalloxid-Pulver;
das dadurch gekennzeichnet ist, dass das erste Metalloxid-Pulver (a) einen d₅₀-Wert von 0,2-12 µm aufweist; und
das zweite, nanoskalige Metalloxid-Pulver (b) einen d₅₀-Wert von 10 bis 300 nm aufweist, wobei das Verhältnis der d₅₀-Werte von (a) zu (b) bei max. 40 zu 1 liegt. Im Stand der Technik sind derartige Metalloxid-Pulverkombinationen wie folgt untersucht worden. M. Moskovits, B.G. Ravi und R. Chaim untersuchten in NanoStructered Materials, Vol.11. No. 2, pp 179-185 ein bimodales Pulver, dessen Nanokomponente eine durchschnittliche Partikelgröße von 10 nm und deren Basiskomponente eine durchschnittliche Partikelgröße von 430 nm aufwies. Bei einem Größenverhältnis beider Komponenten von über 40 und besonders bei einer so feinen Nanokomponente ist die Herstellung einer homogenen Pulvermischung mit vertretbarem technischen Aufwand nur begrenzt möglich. Legt man eine ideale kugelige Gestalt der Basiskomponente zu Grunde, so kann die Optimierung der Packungsdichte nur durch große Agglomerate der nanoskaligen Komponente erreicht werden, wodurch faktisch keine Nanokomponente mehr vorliegt. Von P. Bowen et al., Ceramic Transactions (1998), 211-218, sind bimodale γ-Al₂O₃-Pulver auf ihr Verdichtungsverhalten untersucht worden, wobei die Formgebung durch Schlickerguss oder kalt isostatisches Pressen erfolgte. Die Partikelgröße des gröberen Pulvers betrug 1 µm, während die des Nanopulvers 70-120 nm betrug. Nach dem Sintern fand sich eine Korngröße von ca. 1 µm. Bei den erfindungsgemäßen bimodalen Al₂O₃-Pulvern bzw. Al₂O₃-Binder-Verbünden wurde als der gröbere Bestandteil vorzugsweise allotrophe Modifikationen des Al₂O₃ verwendet.
Möglich ist auch die Verwendung von Übergangstonerden sowie von Mischtypen oxidischer, oxidhydratischer Zusammensetzung, die auch Hydroxylgruppen und unterschiedlich chemisch gebundenes Wasser enthalten können.
Bevorzugt werden jedoch alpha- und gamma-Tonerden benutzt.
Die Ergebnisse liefern kein eindeutiges Bild. Zwar liefert eine kaltisostatische Verdichtung die höchste Gründichte, aber auch eine sehr geringe Sinterdichte und war sogar beim monomodalen γ-Al₂O₃-Pulver größer. Eine Verwendung eines derartigen Pulvers als Fräskeramik in der Dentaltechnik scheidet somit aus. Daher sind zwar bimodale Metalloxid-Pulver aus γ-Al₂O₃, bestehend aus einem ersten γ-Al₂O₃-Pulver mit einer durchschnittlichen Korngröße von 1 µm und einem zweiten γ-Al₂O₃-Pulver mit einer durchschnittlichen Korngröße von 70 bis 120 nm, wie sie von Bowen *et al.* beschrieben wurden, von den erfindungsgemäßen bimodalen Metalloxid-Pulvern ausgenommen. Sofern die Verwendung der erfindungsgemäßen Metalloxid-Pulver in Keramiken, insbesondere in Fräskeramiken, bzw. zu ihrer Herstellung sowie ihre Verwendung in Dentalprodukten betroffen sind, können diese auch aus erfindungsgemäßen bimodalen Metalloxid-Pulvern aus γ-Al₂O₃, bestehend aus einem ersten γ-Al₂O₃-Pulver mit einer durchschnittlichen Korngröße von 1 µm und einem zweiten γ-Al₂O₃-Pulver mit einer durchschnittlichen Korngröße von 70 bis 120 nm, hergestellt sein, wobei der Verwendung von α-Al₂O₃-Pulvern der Vorzug gegeben wird.

Das erfindungsgemäße bimodale Metalloxid-Pulver bzw. der bimodale Keramik-Binder-Verbund stellt nunmehr erstmals ein Metalloxid-Pulver zur Verfügung, aus dem sich Grünkörper- bzw. Vorsinterkeramiken herstellen lassen, die vor der End-Sinterung einer Fräsbearbeitung ohne Auftreten von Ausbrüchen oder anderen, durch die spanende Bearbeitung verursachten Fehlern unterworfen werden können und nach der anschließenden End-Sinterung für eine Verwendung in der Dentaltechnik mechanisch ausreichend belastbar sind. Es hat sich gezeigt, dass Keramiken, die aus dem erfindungsgemäßen bimodalen Metalloxid-Pulver hergestellt werden, eine Reihe von hervorragenden Eigenschaften aufweisen:

Die bimodalen Metalloxyd-Pulver zeichnen sich dadurch aus, dass sie besonders gut in Herstellungsprozesse integrierbar sind, sich insbesondere für einen Einsatz in Plasma-Verfahren eignen. Ferner weisen sie überraschend gute mechanische Eigenschaften auf, insbesondere eignen sie sich für eine Fräsbearbeitung.

Diese Keramiken weisen eine gesteigerte Grünkörperfestigkeit auf, so dass die z.B. nach kalt isostatischem Verdichten aus den erfindungsgemäßen bimodalen Metalloxid-Pulvern erhaltenen Grünkörperkeramiken (bzw. nach anderen Verfahren zugänglichen Vorsinterkeramiken) vor und nach der End-Sinterung spanend und ohne Ausbrüche bearbeitet werden können und somit für die Herstellung möglichst endabmaßnaher Dentalkeramiken geeignet sind. Hinzu kommt die Tatsache, dass diese Grünkörperkeramiken bzw. Vorsinterkeramiken bei der End-Sinterung einen geringen Schrumpf von weniger als 15 % aufweisen. Im Gegensatz dazu weisen die im Stand der Technik bekannten, aus herkömmlichen Metalloxid-Pulvern hergestellten Grünkörperkeramiken bzw. Vorsinterkeramiken nach dem End-Sintern einen Schrumpf von ca. 25 % oder mehr auf. Das kann zu einem Verziehen der Keramik führen und erfordert in den Abmessungen größere Fräswerkzeuge. Es hat sich ferner gezeigt, dass die beim abschließenden Sintern anzuwendende Sinterendtemperatur im Falle der aus den erfindungsgemäßen bimodalen Metalloxid-Pulvern herstellbaren Keramiken erheblich unter der für Keramiken aus üblichen Metalloxid-Pulvern notwendigen Sinterendtemperatur liegt. Damit ergeben sich beim Betreiben des Sinterofens geringere Energiekosten, weil zum Sintern weniger hohe Temperaturen notwendig sind und der Sintervorgang weniger Zeit in Anspruch nimmt. Vorteilhaft können bereits im Dentallabor vorhandene Ofentypen für Presskeramik weiterverwendet werden.

Vollkommen überraschend können aus den erfindungsgemäßen bimodalen Metalloxid-Pulvern Keramiken hergestellt werden, die eine so hohe Transluzenz aufweisen, dass sich bezüglich der ästhetischen Gestaltung für den Dentaltechniker ganz neue Gestaltungsspielräume ergeben. In keinem Fall lag die Transluzenz eines mit der spez. Nanokomponente versehenden Keramikkörpers unter der des Grundpulvers, wo hingegen bimodale Mischungen unter Verwendung von flammpyrolytisch oder mittels Sol-Gel-Verfahren hergestellten Nanokomponenten stets opaker waren als das Grundpulver. Auch eine Verwendung als Optokeramik erscheint somit möglich.

Die aus erfindungsgemäßen bimodalen Metalloxid-Pulvern zugänglichen Keramiken weisen ferner gegenüber bislang bekannten Keramiken aus Metalloxid-Pulvern ohne nanoskaligen Anteil bei gleichen Sinterbedingungen eine erhöhte mechanische Festigkeit auf, was sich vorteilhaft auf die Lebensdauer der Keramiken auswirkt.

Das erfindungsgemäße bimodale Metalloxid-Pulver umfasst ein erstes Metalloxid-Pulver (a) mit einem d₅₀-Wert von 0,2-12 µm und ein zweites, nanoskaliges Metalloxid-Pulver (b) mit einem d₅₀-Wert von 10 bis 300 nm. Es ist möglich, dass das erste Metalloxid-Pulver von einem anderen Metalloxid als das zweite, nanoskalige Metalloxid-Pulver gebildet wird. Bevorzugt werden beide Metalloxid-Pulver (a) und (b) von demselben Metalloxid gebildet. Die Metalloxide sind vorzugsweise aus der Gruppe ausgewählt, die undotiertes ZrO₂, mit CeO₂, CaO, MgO, Sc₂O₃ und Y₂O₃ -dotiertes ZrO₂, sowie TiO₂ und Al₂O₃ enthalten. Besonders bevorzugt ist Y₂O₃ -dotiertes ZrO₂.

Bei den ersten Metalloxid-Pulvern (a) handelt es sich um handelsübliches, kommerziell erhältliches Metalloxid-Pulver z.B. der Fa. Tosoh, Alcoa, Auer-Remy, alusuisse martinswerk, Sumitomo oder Zirconia sales. Üblicherweise ist es mit einem weiteren Metalloxid (Y₂O₃) stabilisiert. Das weitere Metalloxid-Pulver liegt vorzugsweise in einer Menge von 0,5 bis 12 mol-% - bezogen auf die Gesamtmenge an erstem Metalloxid (a) - vor. Als besonders geeignete Stabilisatoren haben sich neben Calciumoxid (CaO) vor allem Magnesiumoxid (MgO) in einer Menge von 7 bis 12 mol-%, insbesondere etwa 9 mol-% MgO bzw. Scandiumoxid (Sc₂O₃), Ceroxid (CeO₂) bzw. Yttriumtrioxid (Y₂O₃) in einer Menge von 1 bis 5 mol-%, insbesondere etwa 3 mol-% Y₂O₃ erwiesen.

Das zweite, nanoskalige Metalloxid-Pulver (b) kann unstabilisiert oder mit einem weiteren Metalloxid stabilisiert sein. Geeignete Stabilisatoren sind u.a. CaO, Sc₂O₃, CeO₂, MgO und insbesondere Y₂O₃. Das weitere Metalloxid-Pulver liegt vorzugsweise in einer Menge von 0,5 bis 12 mol-% - bezogen auf die Gesamtmenge an zweitem, nanoskaligem Metalloxid-Pulver (a) - vor. Das bevorzugte Yttriumtrioxid (Y₂O₃) liegt insbesondere in einer Menge von 1 bis 5 mol-%, besonders bevorzugt etwa 3 mol-% Y₂O₃, vor. Als weitere nanoskalige Metalloxide können auch Al₂O₃ und TiO₂ eingesetzt werden.

Die zur Herstellung der erfindungsgemäßen bimodalen Metalloxid-Pulver verwendeten nanoskaligen Metalloxid-Pulver (b) sind nach allgemein üblichen Syntheseverfahren zugänglich. So können Metalloxid-Pulver z.B. auf verschiedenen chemischen Wegen mittels Sol-Gel-Synthese hergestellt werden. Ein Verfahren ist die Mikroemulsionstechnik nach G. Rinn und H. Schmidt in Ceramic Powder Processing Science (Proceedings of the second International Conference 12-14.10.1988). Weitere Möglichkeiten bieten Y.T. Moon, D.K. Kim, C.H. Kim in J. Am. Ceram. Soc., 78 [4] 1103-106, J.D. Mackenzie in Ultrastructure Processing of Ceramics, Glasses and Composites,1984, S. 15-26, E.A. Barringer und H.K.Bowen in J. Am. Ceram. Soc. 1982, S.199-201, E. Matijevic in Acc. Chem. Res., 1981, S.22-29, Fegley und Barringer in Mat. Res. Soc. Proc. , 1984, S. 187-197. Alternativ kann die Metallsalzsole durch Flammpyrolyse gemäß S. Begand und S. Ambrosius in DKG, S. D12-D16, 1998 und in Chemie Ingenieur Technik, S. 746-749; 1998 nanoskalige Metalloxidpulver liefern. Letztlich können die nanoskaligen Metalloxid-Pulver auch nach einem Plasmasynthese-Verfahren - basierend auf DE 3339490 A1 - erzeugt werden.

Überraschenderweise hat sich gezeigt, dass sich insbesondere bei der Zugabe von plasmasynthetisch hergestelltem nanoskaligen Metalloxid-Pulver, bevorzugt ZrO₂ und Y₂O₃ -dotiertes ZrO₂, besonders gute Ergebnisse, d.h. besonders geringer Schrumpf, hohe Sinterdichte, hohe Biegefestigkeit, hohe Transluzenz usw. der Keramik, erzielen lassen.

Weiterhin ist es bevorzugt, dass das zweite, nanoskalige Metalloxid-Pulver (b) eine durchschnittliche Partikelgröße von 5 bis 70 nm, insbesondere von 14 bis 56 nm und bevorzugt von 40 bis 50 nm aufweist.

Grundsätzlich ist der Gehalt des erfindungsgemäßen bimodalen Metalloxid-Pulvers an dem zweiten, nanoskaligen Metalloxid-Pulver (b) im Hinblick auf die oben dargelegten und wünschenswerten Eigenschaften der aus ihm hergestellten Keramiken nicht nach oben oder unten beschränkt. Es hat sich allerdings gezeigt, dass ein besonders geringer Schrumpf, eine besonders gute Bearbeitbarkeit des Grünkörpers, eine hohe Gewährleistung des isotropen Schrumpfens und eine möglichst hohe Transparenz der Keramiken bei gleichzeitiger hoher mechanischer Belastbarkeit dann erzielt werden, wenn das erfindungsgemäße bimodale Metalloxid-Pulver 5 bis 30 Gew.-%, insbesondere 10 bis 25 Gew.-% und bevorzugt etwa 20 Gew.-% des zweiten, nanoskaligen Metalloxid-Pulvers (b) (bezogen auf das Gesamtgewicht des bimodalen Metalloxid-Pulvers) umfasst.

Die besten Ergebnisse wurden mit einem bimodalen ZrO₂-Metalloxid-Pulver erzielt, das als nanoskaliges Metalloxid-Pulver (b) mit 3 mol-% Y₂O₃ stabilisiertes ZrO₂, welches nach einem Plasmasynthese-Verfahren hergestellt worden ist, in einer Menge von etwa 20 Gew.-% (bezogen auf das Gesamtgewicht des bimodalen Metalloxid-Pulvers) enthält.

Die erfindungsgemäßen bimodalen Metalloxid-Pulver sind ausgehend von ihren Einzelbestandteilen in herkömmlicher Weise zugänglich. Bevorzugt werden sie derart hergestellt, dass
(A) das erste Metalloxid-Pulver (a) und das zweite, nanoskalige Metalloxid-Pulver (b) miteinander gemischt werden; und
(B) das in Schritt (A) gebildete Gemisch einer Granulation unterworfen wird.

Alternativ können die erfindungsgemäßen bimodalen Metalloxid-Pulver auch nach einem Verfahren hergestellt werden, in dem
(A') das erste Metalloxid-Pulver (a) einer Granulation unterworfen wird; und
(B') das in Schritt (A') gebildete Granulat mit dem zweiten, nanoskaligen Metalloxid-Pulver (b) gemischt wird.

Dabei kann das Granulat zunächst vorverdichtet, dann aufgemahlen und anschließend erneut granuliert und entverdichtet wird.

Die Mischung (A) bzw. (B') kann sowohl trocken als auch in Gegenwart eines geeigneten organischen Solvens, z.B. einem Alkohol wie Ethanol, erfolgen.
Durch Zusatz geeigneter Oberflächenmodifikatoren (u.a. Tenside, z.B. Tegotens T826) erfolgt eine verbesserte Desagglomeration auf Primärteilchengröße und eine für die Weiterverarbeitung und Produktqualität wichtige chemische Modifikation der Partikeloberflächen.
Üblicherweise wird unter Rühren für etwa 2 bis 16 h, insbesondere 8 bis 12 h, besonders bevorzugt etwa 10 h, gemischt.

Ein weiterer Gegenstand der vorliegenden Erfindung ist eine Keramik mit bimodaler Korngrößenverteilung, herstellbar aus einem erfindungsgemäßen bimodalen Metalloxid-Pulver, das umfasst
(a) ein erstes Metalloxid-Pulver (a) mit einem d₅₀-Wert von 0,2-12 µm und
(b) ein zweites, nanoskaliges Metalloxid-Pulver mit einem d₅₀-Wert von 10 bis 300 nm mit
(c) einem Größenverhältnis der d₅₀-Werte von (a) zu (b) von max. 40 zu 1

Die aus den erfindungsgemäßen bimodalen Metalloxid-Pulvern zugänglichen Keramiken weisen in der Regel eine bimodale Korngrößenverteilung auf, wobei
(1) eine erste Phase ein Metalloxid mit einer durchschnittlichen Korngröße von mindestens 250 nm umfasst; und
(2) eine zweite Phase ein Metalloxid mit einer durchschnittlichen Korngröße von 25 bis 250 nm umfasst.

Die erfindungsgemäßen Keramiken sind vorzugsweise unter anderem Grünkörper- oder Vorsinterkeramiken; besonders bevorzugt handelt es sich bei den erfindungsgemäßen Keramiken um Fräskeramiken. Aufgrund ihres geringeren Schrumpfens können diese verdichteten Keramiken auch in bereits bestehenden Frässystemen, insbesondere Dentalfrässystemen, welche bislang nur fertig gesinterte Keramiken, also Keramiken, die einer End-Sinterung unterzogen wurden, gefräst haben, spanend bearbeitet werden, bevor sie einer End-Sinterung unterzogen werden. Für die Zwecke der Dentaltechnik können diese Keramiken anschließend zu einem Dentalkeramikprodukt ausreichender Dimension, etwa einer Zahnkrone oder einer Zahnbrücke, gesintert werden. Selbstverständlich können die erfindungsgemäßen Keramiken auch zunächst einer End-Sinterung unterzogen werden, bevor sie weiterverarbeitet werden. Auch die Herstellung einer endgesinterten, porösen Keramik, die einer Infiltration unterzogen werden kann, wird durch die erfindungsgemäße Keramik verbessert, da der poröse Gerüstwerkstoff verbesserte mechanische Eigenschaften aufweist.

Üblicherweise werden die erfindungsgemäßen Grünkörperkeramiken bzw. Vorsinterkeramiken nach an sich bekannten Verfahren dadurch hergestellt, dass das nach den oben beschriebenen Verfahren erhältliche bimodale Metalloxid-Pulver
(C) kalt isostatisch endverdichtet bzw. zunächst vorverdichtet und schließlich endverdichtet und
(C') einer Vorsinterung unterworfen wird [Sintertemperatur 300-1200°C; Sinterdauer 0,5-8 h].

Die kalt isostatische Verdichtung des erfindungsgemäßen bimodalen Metalloxid-Pulvers erfolgt dabei z.B. chargenweise nach dem sog. Wet-Bag-Verfahren in einer CIP-Anlage der Fa. Phi Technologies unter einem Pressdruck von 200 bis 1000 MPa, bevorzugt etwa 300 MPa. Alternativ kann, besonders unter Berücksichtigung der Herstellung von Grünkörpern in großen Stückzahlen, die Verdichtung auch durch kaltisostatische Verdichtung nach dem Dry-Bag-Verfahren oder uniaxial erfolgen.

Dabei kann das Granulat zunächst vorverdichtet, dann aufgemahlen und anschließend erneut granuliert und entverdichtet wird.

Insbesondere ist auch eine Vorverdichtung mit anschließender Aufmahlung des Grünkörpers und einer abschließenden Endverdichtung möglich. Des Weiteren ist auch eine Verarbeitung durch HIP (hot isostatic pressing) möglich. Die auf diese Weise erhaltene Keramik kann dann in einem weiteren Verfahrensschritt (D) einer Sinterung unterzogen werden, bevor eine Weiterverarbeitung erfolgt. Alternativ und besonders bevorzugt wird der verdichtete Grünkörper in einem Verfahrensschritt (E) einer Fräsbearbeitung unterzogen, bevor in einem weiteren Schritt (D') die erhaltene gefräste Keramik einer Sinterung unterworfen wird. Die Sinterung erfolgt dabei in üblichen Sinteröfen, z.B. Bodenladeröfen, und zwar bei Temperaturen von 900 °C bis 1700°C, bevorzugt bei etwa 1300°C; die Sinterdauer beträgt üblicherweise etwa 0,5-20h, bevorzugt 1-4h. Aufgrund der besonderen Eigenschaften der erfindungsgemäßen bimodalen Metalloxid-Pulver, aus denen die erfindungsgemäßen Keramiken zugänglich sind, kann die Bearbeitung bei Verwendung in der Dentaltechnik sehr weitgehend endabmaßnah erfolgen.

Die erfindungsgemäßen Keramiken finden demnach vor allem Verwendung als Fräskeramik, insbesondere als Dentalfräskeramik, ohne jedoch auf diese technische Verwendung beschränkt zu sein. Weitere Verwendungsgebiete sind Bio- und Medizintechnik, sowie generell das Gebiet der technischen Keramiken in der Feinwerktechnik sowie dem Maschinen- und Automobilbau. Aus den erfindungsgemäßen Keramiken herstellbare Dentalkeramikprodukte sind demgemäß ebenfalls Gegenstand der vorliegenden Erfindung. Aus den genannten Eigenschaften der erfindungsgemäßen Keramiken ergibt sich ihre Eignung als Dentalmaterial oder daraus geformtes Dentalprodukt oder als Bestandteil von Dentalmaterial oder von daraus geformtem Dentalprodukt. Bevorzugte Dentalprodukte sind dabei Zahnwurzelkonstruktionen, z.B. Zahnwurzelaufbauten oder Zahnwurzelstifte, oder Zahnbrücken oder Zahnkronen, insbesondere Gerüstkeramiken und Implantatwerkstoff. Die hohe Transluzenz der Keramiken der vorliegenden Erfindung gestattet auch ihren Einsatz als Optokeramik.

Ein weiterer Gegenstand der vorliegenden Erfindung ist daher ein Dentalkeramikprodukt, umfassend eine erfindungsgemäße Keramik.

Nachfolgend wird die Erfindung anhand von Beispielen näher erläutert, ohne dass der Schutzumfang der Erfindung durch sie beschränkt werden soll. Die nachfolgenden Beispiele beinhalten bevorzugte Ausführungsformen und zweckmäßige Weiterbildungen der Erfindung. Weitere Weiterbildungen und Ausgestaltungen der Erfindung sind in den Unteransprüchen enthalten.

### Beispiele

### Vorbemerkung

Die verwendeten Materialien sind kommerziell bzw. durch bekannte Herstellungsverfahren erhältlich.

Korngrößen wurden vor mittels Laserbeugung und nach dem Sintern mittels Rasterelektronenmikroskop bestimmt; die Bestimmung des Sinterschrumpfens erfolgte durch Vermessung der drei Raumachsen und der Raumdiagonalen des quaderförmigen Grün- und Sinterkörpers. Die Bestimmung der Grün- und Sinterdichte erfolgte nach dem Archimedes-Prinzip, die Bestimmung der 3-PunktBiegefestigkeit erfolgte gemäß den Vorgaben der Dentalnorm EN ISO 6872.

### Beispiel 1 (Verqleichsbeispiel)

Ein ZrO₂-Pulver, stabilisiert mit 3 mol-% Y₂O₃, mit einer durchschnittlichen Partikelgröße von 620nm wurde bei 300 MPa min kalt isostatisch verdichtet. Die Gründichte dieses Ausgangspulvers lag bei durchschnittlich 2,69 g / cm³. Der verdichtete Grünkörper wurde in einem Bodenladerofen BL-1801 von Kendro unter folgenden Bedingungen gesintert:
1. Entbinderung: 700 °C
2. Sinterung:1500°C

Die 3-Punktbiegefestigkeit betrug durchschnittlich 1149 MPa.
Der Sinterschrumpf betrug durchschnittlich 24,7 %, die Sinterdichte lag bei 6,03 g/cm³_{.}

### Beispiel 2 (Herstellung von nanoskaligem ZrO₂-Pulver durch ein plasmachemisches

### Syntheseverfahren)

Die Herstellung von nanoskaligem ZrO₂-Pulver erfolgte durch die Zugabe von 30-40 µm großer Partikel der Reinmetalle oder hochflüchtige Metallverbindungen, wie z.B. Chloride direkt in ein Niedertemperaturplasma, welches mit Hilfe von HF- bzw. UHF-Plasmatronen erzeugt wird und ein großes Plasmavolumen und eine kleine Strömungsgeschwindigkeit (hohe Kontaktzeit) aufweist, gegeben. 1757g ZrCl₄ und 190g YCl₃ • 6 H₂O werden bei 3000-7000 K verdampft und ergeben theoretisch 1 kg nanoskaliges ZrO₂ (stabilisiert mit 3 mol-% Y₂O₃), wobei noch die Fraktionierung des Pulvers erfolgen muss. Das Pulver wies eine durchschnittliche Korngröße von 50 nm und einer spezifischen Oberfläche von 26 ± 2 m²/g auf.

### Beispiel 3 (Herstellung eines bimodalen Metalloxid-Pulvers und einer

### entsprechenden Keramik)

Das nanoskalige Metalloxid-Pulver aus Beispiel 2 wurde zunächst mittels einer Ultraschallbehandlung deagglomeriert. Dem oberflächenmodifiziertem Metalloxid-Pulver aus Beispiel 1 wurde mit dem nanoskaligen Metalloxid-Pulver aus Beispiel 2 in einer Menge von 20 Gew.-% (bezogen auf das Gesamtgewicht des bimodalen Metalloxid-Pulvers) in einem Rotationsverdampfer ein Tensid zugesetzt (Verhältnis Pulvermischung zu Lösungsmittel: 1/7). Anschließend wurde dem Gemisch 3 mass-% Binder zugeführt und für 10 h bei 70°C gemischt. Anschließend wurde das Lösungsmittel abgedampft und die Pulvermischung bei 60°C getrocknet. Daraufhin wurde das Pulvergemisch granuliert und kalt isostatisch bei 60 MPa vorverdichtet. Anschließend wurde der Grünkörper gemahlen und bei 300 MPa kalt isostatisch endverdichtet. Der so erhaltene Grünkörper wies eine spezifische Dichte von durchschnittlich 4,14 g/cm³ auf. Die anschließende Sinterung erfolgte wie in Beispiel 1 beschrieben. Die erhaltene Keramik war transluzenter als in Beispiel 1 und wies folgende weitere Eigenschaften auf:

Die 3-Punktbiegefestigkeit betrug durchschnittlich 1473 MPa.

Der Sinterschrumpf betrug 11,8%, die Sinterdichte 6,08 g/cm³.

## Patentansprüche

1. Bimodales Metalloxid-Pulver bzw. bimodaler Metalloxid-Binder-Verbund, umfassend
(a) ein erstes Metalloxid-Pulver (mit und ohne Oberflächenmodifikation); und
(b) ein zweites, nanoskaliges Metalloxid-Pulver (mit und ohne Oberflächenmodifikation),
**dadurch gekennzeichnet, dass**
das erste Metalloxid-Pulver (a) einen d₅₀-Wert von 0,2-12 µm aufweist; und das zweite, nanoskalige Metalloxid-Pulver (b) einen d₅₀-Wert von 10-300 nm aufweist;
wobei das Größenverhältnis der d₅₀-Werte von (a) zu (b) bei max. 40:1 und das Mengenverhältnis von 0,1 : 99,9 bis zu 99,9 : 0,1 liegt, wobei das Metalloxid aus der Gruppe ausgewählt ist, die Hf O₂, TiO₂ und Al₂O₃ umfasst, wobei bimodale Metalloxid-Pulver aus γ-Al₂O₃, bestehend aus einem ersten γ-Al₂O₃-Pulver mit einer durchschnittlichen Korngröße von 1 µm und einem zweiten γ-Al₂O₃-Pulver mit einer durchschnittlichen Korngröße von 70 bis 120 nm ausgenommen sind.

2. Bimodales Metalloxid-Pulver nach Anspruche 1, **dadurch gekennze ichnet**, dass das zweite, nanoskalige Metalloxid-Pulver (b) nach einem Plasmasynthese-Verfahren hergestellt ist.

3. Bimodales Metalloxid-Pulver nach einem der Ansprüche 1 oder 2, **dadurc hgekennzeichnet, dass** das zweite, nanoskalige Metalloxid-Pulver (b) eine durchschnittliche Korngröße von 10 bis 200 nm, insbesondere von 15 bis 100 nm und bevorzugt von 40 bis 50 nm aufweist.

4. Bimodales Metalloxid-Pulver nach einem der Ansprüche 1 bis 3, **dadurch gekennzeichnet, dass** das bimodale Metalloxid-Pulver 5 bis 30 Gew.-%, insbesondere 10 bis 25 Gew.-% und bevorzugt etwa 20 Gew.-% des zweiten, nanoskaligen Metalloxid-Pulvers (b) (bezogen auf das Gesamtgewicht des bimodalen Metalloxid-Pulvers) umfasst.

5. Keramik aus Metalloxid-Pulver mit bimodaler Korngrößenverteilung, herstellbar aus einem bimodalen Metalloxid-Pulver, das umfasst
(a) ein erstes Metalloxid-Pulver mit einem d₅₀-Wert von 0,2-12 µm und
(b) ein zweites, nanoskaliges Metalloxid-Pulver mit einem d₅₀-Wert von 10 bis 300 nm.
wobei das Größenverhältnis der d₅₀-Werte von (a) zu (b) bei max. 40:1 und das Mengenverhältnis von 0,1 : 99,9 bis zu 99,9 : 0,1 liegt.

6. Keramik aus Metalloxid-Pulver mit bimodaler Korngrößenverteilung, umfassend zwei oder mehr Phasen, **dadurch gekennzeichnet, dass**
(1) eine erste Phase ein Metalloxid mit einer durchschnittlichen Korngröße von mindestens 250 nm umfasst; und
(2) eine zweite Phase ein Metalloxid mit einer durchschnittlichen Korngröße von 25 bis 250 nm umfasst.

7. Keramik nach einem der Ansprüche 5 oder 6, **dadurch gekennzeichnet, dass** sie eine Grünkörper- , eine Vorsinter- oder eine Fräskeramik ist.

8. Verfahren zur Herstellung von Grünkörperkeramiken bzw. Vorsinterkeramiken, **dadurch gekennzeichnet, dass** ein bimodales Metalloxid-Pulver nach einem der Ansprüche 1 bis 4
(C) kalt isostatisch (uniaxial) endverdichtet bzw. zunächst vorverdichtet und abschließend einer Endverdichtung und
(C') einer Vorsinterung bei einer Sintertemperatur von 300 bis 1100°C und einer Sinterdauer von 0,5 bis 8 h unterworfen wird.

9. Verfahren zur Herstellung einer Keramik aus einem bimodalen Metalloxid-Pulver nach einem der Ansprüche 1 bis 4, **dadurch gekennzeichnet, dass** das bimodale Metalloxid-Pulver
(C) kalt isostatisch verdichtet bzw.
(C') einer Vorsinterung unterworfen wird, und
(D) die in Schritt (C) erhaltene Grünkörperkeramik bzw. die in Schritt (C') erhaltene Vorsinterkeramik einer Sinterung unterzogen wird.

10. Verfahren zur Herstellung einer Fräskeramik aus einem bimodalen Metalloxid-Pulver nach einem der Ansprüche 1 bis 4, **daduch gekennzeichnet, dass** das bimodale Metalloxid-Pulver
(C) kalt isostatisch verdichtet bzw.
(C') einer Vorsinterung unterworfen wird;
(E) die in Schritt (C) erhaltene Grünkörperkeramik bzw. die in Schritt (C') erhaltene Vorsinterkeramik einer Fräsbearbeitung unterzogen wird; und
(D') die in Schritt (E) erhaltene gefräste Keramik einer Sinterung unterworfen wird.

11. Verfahren nach einem oder mehreren der Ansprüche 8 bis 10, **dadurch gekennzeichnet, dass** bimodales Pulver vermischt werden und dass in einem Rotationsverdampfer anschließend ein Abdampfen von Lösungsmittelbestandteilen erfolgt.

12. Verwendung eines bimodalen Metalloxid-Pulvers nach einem der Ansprüche 1 bis 4 zur Herstellung einer Keramik, insbesondere einer Fräskeramik oder eine Dentalfräskeramik.

13. Verwendung einer Keramik nach einem der Ansprüche 5 bis 7 als Fräskeramik, insbesondere als Dentalfräskeramik.

14. Verwendung von nanoskaligem Metalloxid-Pulver (b) mit einer durchschnittlichen Korngröße von 1 bis 200 nm zur Herstellung eines bimodalen Metalloxid-Pulvers nach einem der Ansprüche 1 bis 4 und/oder einer Keramik nach einem der Ansprüche 5 bis 7.

15. Verwendung einer Keramik nach einem der Ansprüche 5 bis 7 als Dentalmaterial oder daraus geformtes Dentalprodukt oder als Bestandteil von Dentalmaterial oder von daraus geformtem Dentalprodukt.
